# EUROPEAN PATENT APPLICATION

(11) **EP 1 470 807 A1**
(43) Date of publication of application: **27.10.2004**
(21) Application number: 02792017.2
(22) Date of filing: 26.12.2002
(51) Int. Cl.: A61F 5/44, A61F 5/453, A61F 5/455

(54) **ABSORBING MATERIAL PRODUCT, INNER BAG AND UNDERPANTS HAVING THEM**

(30) Priority: 28.12.2001 JP 2001401488
(71) Applicant: Japan Absorbent Technology Institute, Chuo-ku, Tokyo 103-0007 (JP)
(72) Inventor: SUZUKI, M. Japan Absorbent Technology Institute, Chuo-ku, Tokyo 103-0007 (JP); MORIYA, Reiko Japan Absorbent Technology Institute, Chuo-ku, Tokyo 103-0007 (JP); SUGIYAMA, Katsuhiko c/o Oji Paper Co., Ltd., Kasugai-shi, Aichi 486-0834 (JP); TAKEMATSU, Satomi c/o Oji Paper Co., Ltd., Chuo-ku, Tokyo 104-0061 (JP)
(74) Representative: Gervasi, Gemma, Dr.
(86) International application number: PCT/JP2002/013644
(87) International publication number: WO 2003/057096

(57) **Abstract**

An absorptive product of the present invention comprises an absorptive body capable of absorbing and holding liquid and a flexible and liquid-impermeable trapping portion which temporarily traps the liquid which is to be absorbed by this absorptive body. Then, the underpants comprising the fitting section to which this absorptive product is detachably fit are made. For this absorptive body, a high liquid absorbing resin of 50 to 95 weight % is used.

## Description

### Technical Field

The present invention relates to a non-bulky and practical absorptive product, inner bag and underpants equipped with these.

### Background Art

The absorptive body, which has the role of absorbing and holding a liquid such as urine in various types of conventional absorptive product, with an absorptive body interpositioned between a liquid-permeable top sheet and a liquid-impermeable back sheet, such as baby diapers, adult diapers, women's sanitary products, light-case to medium-case continence pads, and pets' litter-box filler, is primarily composed of an absorbent core which is a mixture of fluffy pulp and SAP (super absorbent polymer) and a core-covering material such as tissue that wraps this absorbent core. The absorptive products are made by combining the above-mentioned top sheet, back sheet, gather element, binding mechanism, etc. with this absorptive body.

From the standpoint of resource saving, logistics cost reduction, improvement of shelf-efficiency in retail shops, etc. the so-called super-thin-type or ultra-super-thin-type, light and compact absorptive products meet the markets' demand, and these are becoming the mainstream products. The most significant point necessary to realize these super-thin-type absorptive products is downsizing and reducing the weight of the absorptive body which takes up the majority of the weight and volume of the absorptive products. In other words, making thin-type absorbent cores made of the mixture of fluffy pulp and SAP is realized by increasing the relative content of SAP or using the SAP in sheet form in place of a mixture.

Even when the thin-type absorptive products are made possible by increasing the relative content of SAP in the absorptive body or using the SAP in sheet form in place of a mixture, there is a limit for the SAP content ratio, and conventionally the limit was only as high as 50 to 60 weight %. This limit is caused by SAP's gel-blocking phenomenon and its absorption property, and SAP's absorption speed is particularly problematic, though it has high absorbing and holding capability, making it a slow starter.

For instance, the speed of urinary drainage varies depending on the person, gender, age and so on, but the fastest example would be that 100 cc of urine is discharged in about 10 seconds. In comparison to this speed, it takes SAP, even the kind whose absorption speed is improved, more than 30 seconds at least to reach the state of fully displaying its absorbent capability after contacting the liquid. Due to this great speed difference between the speed of urinary drainage and the speed of liquid absorption by SAP, which makes up a part of the absorptive body, a large amount of urine ends up accumulating unabsorbed in the absorptive product during the phase right after the urine is discharged. Free movement of this unabsorbed urine in the absorptive product becomes a major cause of urinal leakage from the absorptive products.

Due to this, in general practice, by interpositioning between the surface of the absorptive body with high SAP content and the top sheet, the bulky non-woven web which is called the "acquisition layer" or "transfer layer" or the partially cross-linked fluff-pulp layer with a great restorative force of resilience which is called the "curly fiber", free movement of urine inside the absorptive product was restrained by having these functional elements temporarily trap the urine, and by eventually having SAP gradually absorb the urine held in these functional elements.

Therefore, the higher the SAP content in the absorptive body becomes, the thicker the laminated acquisition layer or transfer layer needs to be formed. This, however, led in the opposite direction, where making a thin-type of absorptive body was inhibited and on top of that, an increase in production cost was incurred.

### Disclosure of Invention

An object of the present invention is to provide an extremely thin and low-volume-to-weight absorptive product which enables the use of an absorptive body of set SAP content ratio of 50 to 95 weight %, or preferably 80 to 90 weight %, the method of producing the same, the inner bag used in the same, and the method of producing it.

The other object of the present invention is to provide extremely thin and non-bulky underpants which enable the fitting of the absorptive body of a set SAP content ratio of 50 to 95 weight %, or preferably 80 to 90 weight %.

Preferred forms of the above-described invention are given below.

The absorptive product of the present invention comprises an absorptive body that can absorb and hold liquid and a flexible and liquid-impermeable trap portion that temporarily traps the liquid which is to be absorbed by this absorptive body.

In the present invention, when a liquid such as urine is discharged in a large volume, the liquid which cannot be instantaneously absorbed by the absorptive body is temporarily trapped in the trap portion, and the liquid trapped in the trap portion is gradually absorbed and held by the absorptive body over time.

The method of producing the absorptive product of the present invention is the method of producing the absorptive product comprising the trap portion which has an outer bag, that has a penis-guiding section to guide the penis into it, and the flexible liquid-impermeable inner bag, that is incorporated in this outer bag and holds at least one end of the absorptive body; the inner bag that has the folded portion which can evaginate; and the openings that are formed in the outer bag to allow parts of the folded portion of the inner bag to evaginate out of the outer bag, and the method involves a step to form a pair of cross-directional straight-line crena along both side edges of the single sheet material to make an opening on each side of this single sheet material, a step to fold the said single sheet material in a width-wise direction in such a manner as to include the said pair of crena in the folded portion, and a step to form the trap portion by joining the folded portions that overlap each other along the cross-sectional side edges of the said single sheet material.

The inner bag of the present invention is the inner bag used in the absorptive product of the present invention that has the trap portion which has an outer bag with a penis-guiding section to guide the penis into it, and the flexible liquid-impermeable inner bag incorporated in this outer bag holding at least one end of the absorptive body and the inner bag that has the folded portion which can evaginate, and this inner bag comprises the first pair of folded portion, one each on the right and left, formed by folding the right side and left side of the rectangular sheet toward the center, the second folded portion formed by folding the bottom end of the said sheet upward, the third folded portion formed by folding the top end of this second folded portion downward, and the hanger tape each of whose both ends is joined to the center of this third folded portion and the center of the top part of the said sheet.

In the present invention, in case a large volume of urine is discharged inside the inner bag, the first and the second folded portions located on both sides of the hanger tape evaginate and thus increase the capacity of the inner bag and inhibit the leakage of urine from the inner bag.

The method of producing the inner bag of the present invention consists of a step to form the first folded portion by folding both the right and left sides of the rectangular sheet toward the center, a step to form the second folded portion by folding the bottom end of the said sheet upward, a step to form the third folded portion by folding once again the top portion of the said second folded portion downward, and a step to join each of the both ends of the hanger tape to the center of the said third folded portion and the center of the top part of the said sheet.

The underpants of the present invention have a fitting section to which the absorptive product of the present invention or the absorptive product produced by the method of the present invention is fitted, in such a manner that it is detachable.

The underpants of the present invention comprise an absorptive product of the present invention or the absorptive product produced by the method of the present invention, and a fitting section to which this absorptive product is fitted, in such a manner that it is detachable.

The underpants of the present invention comprise the absorptive body holding section to detachably hold the absorptive body that absorbs and can retain the liquid and the flexible, and a liquid-impermeable trap portion to temporarily trap the liquid that is to be absorbed by this absorptive body.

In the present invention, when a liquid such as urine is discharged in a large volume, the liquid which cannot be instantaneously absorbed by the absorptive body is temporarily trapped in the trap portion, and the liquid trapped in the trap portion is gradually absorbed and held by the absorptive body over time. The used absorptive body can be removed from the absorptive body holding section and replaced with a new absorptive body, enabling repeated use of the underpants.

The underpants of the present invention comprise the absorptive body that absorbs and can retain the liquid, the absorptive body holding section to detachably hold this absorptive body and the flexible and liquid-impermeable trap portion to temporarily trap the liquid that is to be absorbed by this absorptive body.

In the present invention, when a liquid such as urine is discharged in a large volume, the liquid which cannot be instantaneously absorbed by the absorptive body is temporarily trapped in the trap portion, and the liquid trapped in the trap portion is gradually absorbed and held by the absorptive body over time. The used absorptive body can be removed from the absorptive body holding section and replaced with a new absorptive body, enabling repeated use of the underpants.

In accordance with the absorptive product of the present invention, because it comprises the absorptive body that absorbs and can retain the liquid and the flexible and the liquid-impermeable trap portion to temporarily trap the liquid that is to be absorbed by this absorptive body, it is possible to have the trap portion to temporarily trap the liquid which cannot be fully absorbed by the absorptive body in a short time, and then have the absorptive body absorb the liquid trapped in this trap portion gradually over time, leading to maximizing the absorptive body's absorption capability. As a result, it also becomes possible to make thinner absorptive products with improved comfort for wearers by simplifying the structure of the absorptive body itself.

If the trap portion is formed with the liquid-impermeable sheet, and the surface of this sheet and at least a part of the absorptive body are made to overlap, it is possible to make the absorptive body absorb the liquid via this sheet.

If an opening facing the surface of the sheet is provided in the trap portion, it is possible to guide the liquid to the trap portion via this opening.

If the opening is extended along the absorptive body in a longitudinal direction, it is possible to have the liquid trapped nearly uniformly in all parts of the trap portion.

If the opening is extended along the absorptive body in a width-wise direction, it is possible to have the liquid trapped nearly uniformly in all parts of the trap portion.

If the absorptive body is positioned to straddle the opening, it is possible to have the absorptive body absorb the liquid trapped in the trap portion via the opening without any special contrivance.

If a continuous aperture is provided in the absorptive body, from the opposite side of the surface of the sheet, facing the opening of the trap portion, even when the liquid is discharged over a short period of time, it is possible to guide it quickly to the trap portion.

If a sheet is provided with a pair of flaps extended along both width-wise side edges of the absorptive body, and a trap portion is formed in each of this pair of flaps, it is possible not only to position the trap portion away from the absorptive body but also to temporarily trap a larger volume of liquid.

If a trap portion is positioned on both width-wise sides of the absorptive body, it is possible to trap temporarily a larger volume of liquid.

If a guiding member (or sometimes called guiding sheet) to guide the liquid trapped in the trap portion to the absorptive body is provided, even when the absorptive body and the trap portion are not in contact, it is possible to ensure that the absorptive body absorbs the liquid trapped in the trap portion.

If the trap portion is formed with the liquid-impermeable sheet, provided with the penis-guiding section to guide the penis into the trap portion, and at least one end of the absorptive body is held in the trap portion, it is possible not only to trap a large volume of urine in the trap portion but also to have the urine absorbed more quickly inside the trap.

If the trap is in form of a pocket holding one end in a longitudinal direction of the absorptive body, it is possible to trap a large volume of liquid in the trap portion.

If the trap portion comprises the outer bag, with a penis-guiding section to guide the penis into it, and the flexible liquid-impermeable inner bag that is incorporated in this outer bag and holds at least one end of the absorptive body, it is possible to give liquid-permeability to the outer bag that contacts the skin of the wearer, thus comfort to the wearer is obtained.

If the inner bag has the folded portion that can evaginate, it is possible to hold a large volume of liquid in the inner bag.

If openings, that are formed in the outer bag to allow parts of the folded portion of the inner bag to evaginate out of the outer bag, are provided, even when a large volume of liquid accumulates temporarily inside the inner bag as a result of the absorptive body's failing to fully absorb the liquid, it is possible for the folded portion to expand and evaginate out of the outer bag through the opening and prevent the liquid from overflowing from the inner bag.

If a dislocation prevention member is provided between the sheet and the absorptive body, to prevent the absorptive body from dislocating against the sheet, it is possible to prevent the absorptive body from dislocating against the sheet.

If a pair of elastic members are provided along the right and left side edges of the sheet, joined in a stretched state to both the right and left side edges of the sheet, when the absorptive product is in use, its close fit to the skin can be improved.

If the absorptive body has a non-woven substrate in a sheet form and there are multiple liquid-absorbing portions coated on this non-woven substrate at prescribed intervals, especially when the absorptive body contains 50 to 95 weight % of SAP, the volume can be made compact, to less than 1/3 compared to such conventional products as thin-type diapers, making possible the provision of various types of absorptive products for babies and adults.

If the absorptive body has a hydrodisintegrative characteristic, disposal "as is", without any kinds of special treatment, will be possible.

In accordance with the method of producing the absorptive product of the present invention, the absorptive product of the present invention can be easily and rationally produced because a pair of cross-directional straight-line crena are formed along both side edges of the single sheet material, to make an opening on each side of this single sheet material, and the single sheet material is folded in the width-wise direction in such a manner as to include the pair of crena in the folded portion, to form the trap portion by joining the folded portions that overlap each other along the cross-sectional side edges of the single sheet material.

In accordance with the inner bag used in the absorptive product of the present invention, it is possible to ensure the formation of the portions that are allowed to evaginate, without making a complicated accordion structure, but by simply folding the rectangular film and by joining with the hanger tape at the predetermined locations.

It is possible to produce the inner bag extremely easily and at low cost, by simply folding the rectangular film and by joining with the hanger tape at the predetermined locations.

Because the absorptive product of the present invention or the absorptive product produced according to the method of the present invention has a fitting section to which the absorptive product of the present invention is fitted, in such a manner that it is detachable, it is possible to remove the used absorptive product from the underpants and fit a new absorptive product to the underpants.

Because the underpants of the present invention have the absorptive product of the present invention or the absorptive product produced according to the method of the present invention, and a fitting section to which this absorptive product is fitted, in such a manner that it is detachable, it is possible to remove the used absorptive product from the underpants and fit a new absorptive product to the underpants.

If the fitting section is provided inside the underpants, it is possible to prevent such problems as the absorptive product's being dislocated from the underpants' prescribed location while in use.

If the underpants are of a plain knit product, a close fit of the absorptive product to the wearer's skin can be ensured.

In accordance with the underpants of the present invention, because they comprise the absorptive body holding section to detachably hold the absorptive body that absorbs and can retain the liquid and the flexible and liquid-impermeable trap portion to temporarily trap the liquid that is to be absorbed by this absorptive body, it is possible to have the trap portion temporarily trap the liquid which cannot be fully absorbed by the absorptive body in a short time, and then have the absorptive body absorb the liquid trapped in this trap portion gradually over time, leading to maximizing the absorptive body's absorption capability. As a result, it also becomes possible to simplify the structure of the absorptive body itself and improve comfort for wearers.

In accordance with the underpants of the present invention, because they comprise the absorptive body that absorbs and can retain the liquid, the absorptive body holding section to detachably hold this absorptive body, and the flexible and liquid-impermeable trap portion to temporarily trap the liquid that is to be absorbed by this absorptive body, it is possible to have the trap portion to temporarily trap the liquid which cannot be fully absorbed by the absorptive body in a short time, and then have the absorptive body absorb the liquid trapped in this trap portion gradually over time, leading to maximizing the absorptive body's absorption capability. As a result, it also becomes possible to simplify the structure of the absorptive body itself and improve comfort for wearers.

If an opening facing the inner surface of the underpants is provided in the trap portion, it is possible to guide the liquid to the trap portion via this opening.

If the opening is extended along the absorptive body in a longitudinal direction, it is possible to have the liquid trapped nearly uniformly in all parts of the trap portion.

If a trap portion is positioned on both sides of the crotch of the underpants, it is possible to trap temporarily a larger volume of liquid.

If a trap portion is positioned on both width-wise sides of the absorptive body, it is possible to trap temporarily a larger volume of liquid.

If an opening into which at least one end of the absorptive body is inserted is provided in the trap portion, it is possible to have the absorptive body absorb the liquid trapped in the trap portion via the opening without any special contrivance.

If the absorptive body has a non-woven substrate in a sheet form and there are multiple liquid-absorbing portions coated on this non-woven substrate at prescribed intervals, especially when the absorptive body contains 50 to 95 weight % of SAP, because it is possible to greatly increase its liquid absorption capability, thinning of the section of the underpants where the absorptive body is held is possible, further improving the comfort for the wearer.

### Brief Description of Drawings

Fig. 1 is a front view of an example of the absorptive product in accordance with the present invention.
Fig. 2 is an oblique perspective figure of the external appearance of one other example of the absorptive product in accordance with the present invention.
Fig. 3 is a longitudinal section view of the center of the example shown in Fig. 2.
Fig. 4 is an oblique perspective figure of the external appearance of an example of the absorptive body in the present invention.
Fig. 5 is a front view of another example of the absorptive product in accordance with the present invention.
Fig. 6 is a front view of yet another example of the absorptive product in accordance with the present invention.
Fig. 7 is a sectional view of the example shown in Fig. 6, taken along the line VII to VII.
Fig. 8 is a front view of a different example of the absorptive product in accordance with the present invention.
Fig. 9 is a sectional view of the example shown in Fig. 8, taken along the line IX to IX.
Fig. 10 is a front view of another different example of the absorptive product in accordance with the present invention.
Fig. 11 is a sectional view of the example shown in Fig. 10, taken along the line XI to XI.
Fig. 12 is a plan view of yet another different example of the absorptive product in accordance with the present invention.
Fig. 13 is a plan view of an example of the absorptive product in accordance with the present invention.
Fig. 14 is a sectional view of the example shown in Fig. 13, taken along the line XIV to XIV.
Fig. 15 is a front view of an example where the absorptive product in accordance with the present invention is applied to the men's continence pad.
Fig. 16 is a longitudinal section view of the center of one other example where the absorptive product in accordance with the present invention is applied to the men's continence pad.
Fig. 17 is a front view of another example where the absorptive product in accordance with the present invention is applied to the men's continence pad.
Fig. 18 is a sectional view of the example shown in Fig. 17, taken along the line XVIII to XVIII.
Fig. 19 is an oblique perspective figure of the external appearance of the inner bag in the example shown in Fig. 17.
Fig. 20 is an illustrative oblique perspective figure of the external appearance of the inner bag shown in Fig.17 in the state where the liquid is temporarily held in it.
Fig. 21 is an oblique perspective figure of the external appearance of the absorptive body in the example shown in Fig. 17.
Fig. 22 is a plan view of the trap sheet in the example shown in Fig. 17 in the opened state.
Fig. 23 is a conceptual rendering of the example shown in Fig. 17 in use.
Fig. 24 is a conceptual rendering of the example where the underpants in accordance with the present invention are applied to the men's briefs.
Fig. 25 is a conceptual rendering of the other example where the underpants in accordance with the present invention are applied to the men's briefs.
Fig. 26 is an inside view of an example of the underpants in accordance with the present invention.
Fig. 27 is an oblique perspective figure of the external appearance of the example where the underpants in accordance with the present invention are applied to the women's panties.
Fig. 28 is a plan view of the crotch of the example shown in Fig. 27 when it is viewed from inside.
Fig. 29 is a sectional view of the example shown in Fig. 28, taken along the line XXIX to XXIX.
Fig. 30 is a plan view of the crotch of the other example where the underpants in accordance with the present invention are applied to the men's briefs, when it is viewed from inside.
Fig. 31 is a sectional view of the example shown in Fig. 30, taken along the line XXXI to XXXI.

### Best Mode for Carrying Out the Invention

In the absorptive product of the present invention, the trap portion may be formed with the liquid-impermeable sheet, and the surface of this sheet and at least a part of the absorptive body may overlap.

In this case, the trap portion may have an opening facing the surface of the sheet, and this opening may be extended along the absorptive body in a longitudinal direction or extended along the absorptive body in a width-wise direction. Also, the absorptive body may be positioned to straddle the opening and in this case the absorptive body may have a continuous aperture penetrating from the opposite side of the surface of the sheet, facing the opening of the trap portion. Furthermore, a sheet may be provided with a pair of flaps extended along both width-wise side edges of the absorptive body, and a trap portion may be formed in each of this pair of flaps.

The trap portion may be positioned on both width-wise sides of the absorptive body.

A guiding member (or a guiding sheet) to guide the liquid trapped in the trap portion to the absorptive body may be further provided.

The trap portion may be formed with the liquid-impermeable sheet, provided with a penis-guiding section to guide the penis into the trap portion, and at least one end of the absorptive body may be held in the trap portion.

The trap portion may comprise the outer bag with a penis-guiding section to guide the penis into it, and the flexible liquid-impermeable inner bag that is incorporated in this outer bag and holds at least one end of the absorptive body. In this case, the inner bag may have a folded portion that can evaginate and, further, it may have openings that are formed in the outer bag to allow parts of the folded portion of this inner bag to evaginate out of the outer bag.

A dislocation prevention member may be further provided between the sheet and the absorptive body, to prevent the absorptive body from dislocating against the sheet.

A pair of elastic members may be further provided along the right and left side edges of the sheet, joined in a stretched state to both the right and left side edges of this applicable sheet.

The absorptive body may have a non-woven substrate in a sheet form and there may be multiple liquid-absorbing portions coated on this non-woven substrate at prescribed intervals. It may contain 50 to 95 weight % of SAP, and may have a hydrodisintegrative characteristic.

In the underpants of the present invention, a fitting section to fit the absorptive product may be provided inside the underpants. The underpants may be of a plain knit product.

In the underpants of the present invention, the trap portion may have an opening facing the inner surface of the underpants. In this case, the opening may be extended along the absorptive body in a longitudinal direction.

The trap portion may be positioned on both sides of the crotch of the underpants, or an opening, into which at least one end of the absorptive body is inserted, may be provided in the trap portion.

The absorptive body may have a non-woven substrate in a sheet form and there may be multiple liquid-absorbing portions coated on this non-woven substrate at prescribed intervals, and it may contain 50 to 95 weight % of SAP.

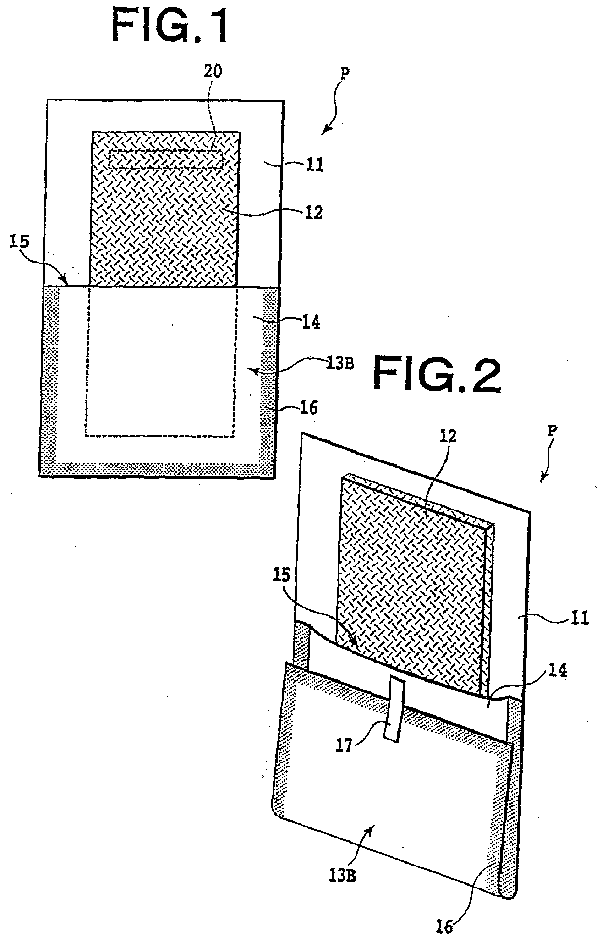

The examples of the absorptive product and the method of producing the same, as well as the underpants using the same, in accordance with the present invention will be explained in further detail with reference to Fig. 1 through Fig. 31. The present invention, however, is not limited to these examples only, in that further combinations of these examples as well as any and all changes and modifications included in the concept of the present invention described in the patent's claims are possible. The present invention, therefore, can be applied as a matter of course to any other technologies which belong to the concept of the present invention.

A front configuration of the first example is shown in Fig. 1. That is, the absorptive product P in the present example comprises a liquid-impermeable backup sheet (11), an absorptive body (12) integrally placed on this backup sheet (11), and a trap sheet (14) forming, together with the backup sheet (11), a flexible and pouched trap portion (13B) in which at least one end (generally going on the lower side when in use) of the absorptive body (12) is held. The other end of the absorptive body (12) in this example protrudes through the opening (15) of the pouched trap portion (13B) and extends to the other end of the backup sheet (11).

The pouched trap portion (13B) formed by the backup sheet (11) and the trap sheet (14) holds at least one end of the absorptive body (12), and depending on the type of use, the pouched trap portion (13B) may be in one case maintained in a sealed condition to protect itself from liquid leakage and in the other case may be provided with an outer-bag-function to allow liquid permeation by concomitantly using the liquid-impermeable inner bag, which will be discussed later.

As a type of use for which the pouched trap portion (13B) needs to be kept in the sealed state, in other words, in the liquid-impermeable state, the first example to be cited may be the case in which the absorptive body (12) to be held in the pouched trap portion (13B) is not covered with a heavy-duty retention sheet such as the top sheet and the backup sheet, and the absorptive material such as SAP or pulp is only wrapped with thin covering material such as tissue paper. In this case, because it is necessary to prevent liquid leakage from the absorptive material, a liquid-impermeable characteristic is given to the pouched trap portion (13B). The second example is the case where it is used with the purpose of urine treatment especially targeted to men. SAP is able to retain a large amount of liquid, but at the same time it has the characteristic of slowly absorbing the liquid, and when the absorptive body (12) that contains a high ratio of SAP with these characteristics is used, SAP's speed of absorbing urine cannot keep up with the speed of urinary drainage, thus requiring a measure to prevent the absorptive body (12) from leaking the urine which is more than it can absorb. In a case like this, the liquid-impermeable pouched trap portion (13B) can be made to function as a flexible container to temporarily hold urine.

As a type of use for which the pouched trap portion (13B), especially the trap sheet (14), is made to have liquid-permeability, the first example is the case where the liquid-impermeable inner bag is concomitantly used with it, and this inner bag is held inside the pouched trap portion (13B). In this case, because the pouched trap portion (13B) has no other functions but to hold the inner bag as an outer bag, it is possible to use a highly-porous or mesh-type trap sheet (14), as well as to construct the trap sheet (14) with such materials as hydrophilic non-woven fabric. The second example is the case where it is used as a female urine pad. In this case also, at least one end (the bottom end) of the trap sheet (14) needs to be made liquid-impermeable to prevent the absorptive body (12) from leaking the urine which is more than it can absorb.

Both the backup sheet (11) and the trap sheet (14) of the present example are made of a liquid-impermeable and flexible PE sheet or a laminated sheet of PE film and non-woven fabric, and a pouched trap portion (13B) with an opening at the other end (top end) of the trap sheet (14) can be formed by overlapping the trap sheet (14) with the backup sheet (11) at one longitudinal end of the latter and making a heat-sealed portion (16) by heat-bonding the outer margin of the backup sheet (11) where the trap sheet (14) overlaps it.

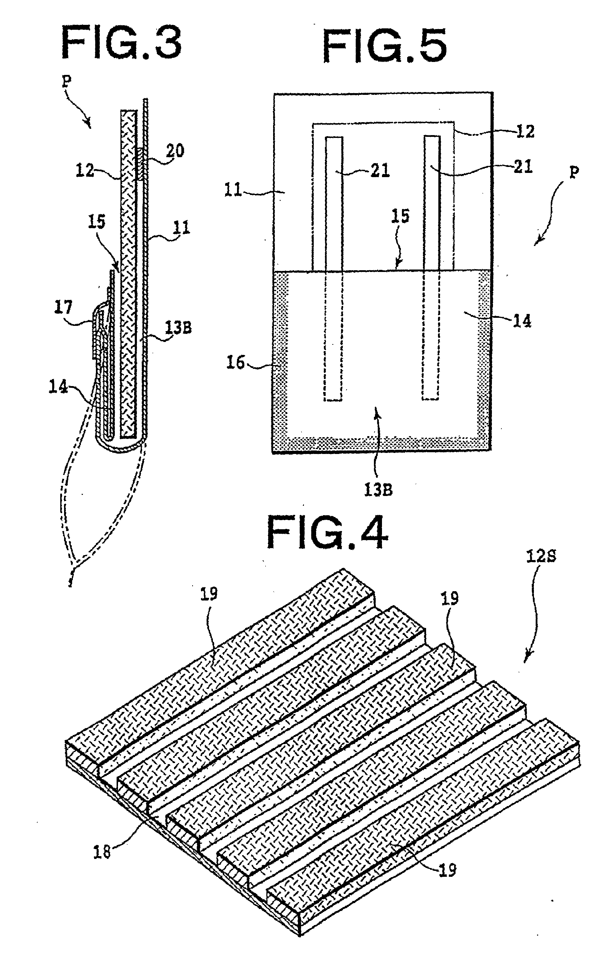

The external appearance of the other example of the present invention, which can increase the capacity of the pouched trap portion (13B) while maintaining the absorptive product P in a compact size, is shown in Fig. 2, and a longitudinal section view of its center is shown in Fig. 3. That is, one end (the bottom end in the Fig.) of the pouched trap portion (13B) is folded upward and its width-wise center and the other end of the trap sheet (14) near the opening (15) of the pouched trap portion (13B) are detachably joined with a temporary tape (17), and in case a large volume of liquid is trapped in the pouched trap portion (13B), due to its weight, the temporary tape (17) may come off from either the backup sheet (11) or the trap sheet (14), causing the pouched trap portion (13B) to evaginate.

If a single synthetic film material is used for both the backup sheet (11) and the trap sheet (14), it is also possible to form the backup sheet (11) and the trap sheet (14) so that they have a pouched trap portion (13B), by folding a long sheet and heat-bondling both cross-sectional side edges of this long sheet's folded portion.

By using a liquid-impermeable backup sheet (11), the absorptive body (12) can be constructed with nothing more than just the ordinary absorptive materials that primarily use such absorptive materials as SAP and wood pulp, and a surface covering sheet such as a liquid permeable top sheet or tissue paper to be used to wrap them and to face the backup sheet (11). Simplification of the structure of the absorptive body (12), as found in this case, allows simplification of the design of the absorptive product P and cost reduction.

For the compact construction of the absorptive product P, and further for the hygienic treatment of urine, it is preferable that the absorptive body (12) is extremely thin and has excellent morphological stability both before and after absorption, so that at least one end of it is smoothly and stably held inside the pouched trap portion (13B), and its shape is stably maintained after the swelling caused by liquid absorption. From this standpoint, the absorptive body (12) used in the present invention should have much higher SAP content ratio than the SAP/pulp ratio seen in ordinary disposable diapers and sanitary napkins, and further, it is preferable that an absorptive body in a sheet-form that can stably maintain its shape, such as the one described in the Japan Patent Application Laid-open Hei 11-34200, is used.

Fig. 4 is an illustrative expression of the external appearance of such an absorptive body in a sheet-form (12S). This absorptive body in a sheet-form (12S) is constructed by making the highly absorbent areas (19), coated, by applying a line-coating technique like curtain-coating, in such a manner as to create multiple band-shapes in parallel with one another on the surface of the base sheet (18) along the longitudinal direction of the base sheet (18), made of non-woven fabric, preferably with bulkiness and air-permeability. In these highly absorbent areas (19), 50 to 95 weight % SAP, preferably 80 to 90 weight %, is contained. The absorptive body in a sheet-form like this (12S) has excellent liquid-dispersing capability, due to the combination of the highly absorbent areas (19) and the areas without it where the base sheet (18) is exposed.

In order not to spoil the expandability of the absorptive body in a sheet-form (12S), it is preferable that no surface-covering sheet such as non-woven fabric, tissue paper, or highly-porous film is used for this absorptive body in a sheet-form (12S) and that at least one end of it is held in the pouched trap portion (13B). As required, however, it can also be folded in a certain configuration and then it can be inserted in the pouched trap portion (13B).

By adopting a so-called flushable material, which can be flushed away in the toilet, for the base sheet (18), and by adopting a bio-degradable amino-acid SAP, it will be possible, as required, to remove the used absorptive body (12) from the pouched trap portion (13B) and then safely dispose of it by flushing it away in the toilet.

In case the depth of the pouched trap portion (13B) is shallow in comparison to the length of the absorptive body (12), in order to prevent dislocation of the absorptive body (12) against the backup sheet (11), a dislocation-prevention member (20) is provided between the backup sheet (11) and the absorptive body (12). In the present example, a hot-melt adhesive is used as the dislocation-prevention member (20), but because the absorptive body (12) does not have to be permanently fixed to the backup sheet (11), it is also possible to allow the absorptive body to be removed from the backup sheet (11) by using an adhesive or a hook and loop fastener. Nothing more than just the prevention of dislocation by friction, using the anti-slip material as the dislocation-prevention member (20), also has some effect. The location, shape and the number of this dislocation-prevention member (20) to be used can be chosen randomly.

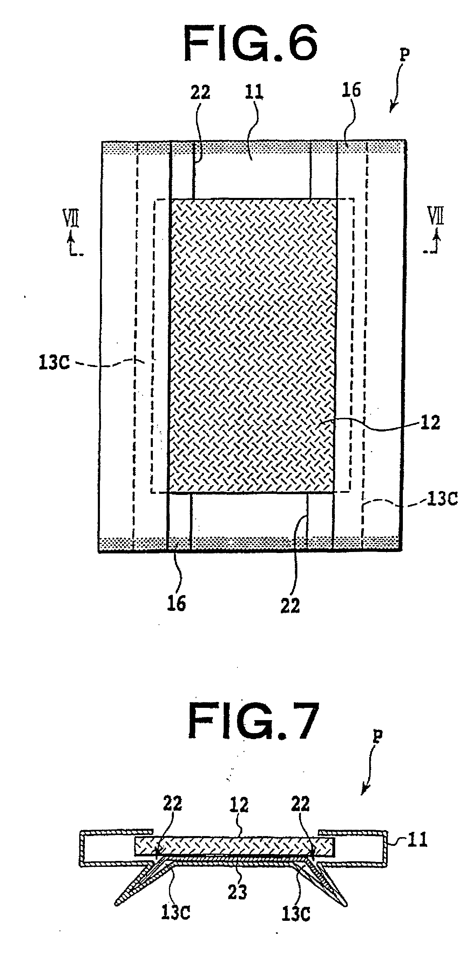

In order to bend the absorptive product P to fit the wearer's contour, it is also effective to deform the absorptive product P with the contractile force of the elastic member (21) that is joined, in the stretched state by about, for instance, 20%, to both side edges in width-wise direction of the backup sheet (11). The other example of the absorptive product P in its opened state is shown in Fig. 5, in which the absorptive body (12) is omitted for the sake of convenience. The elastic member (21) in the present example is placed along both the width-wise side edges of the backup sheet (11), almost throughout its longitudinal length, but it is also acceptable to place the elastic member (21) only on the other end of the backup sheet (11).

If there is no external force applied to the absorptive product P, the elastic member (21) acts to make the width-wise side edges of the backup sheet (11) contract in a longitudinal direction against the other parts. Due to this, when it is put on the wearer's body, both of the width-wise side edges of the absorptive product P are deformed to closely fit the body, preventing leakage of urine from the width-wise side edges.

In the above-mentioned example, gravity is positively used to make the liquid be temporarily trapped in the pouched trap portion (13B). However, depending on the absorption capability of the absorptive body (12), there may be cases where it is difficult to control the liquid's speed of movement and the volume of liquid to be moved. Some other examples of the absorptive product P with the trap portions in a slit form designed based on this knowledge are shown in Fig 6 through Fig. 11 respectively.

The absorptive product P shown in Fig. 6 and its sectional view taken along the line VII to VII, shown in Fig. 7, has 2 trap portions (13C) in a slit form formed in parallel with each other and with some space between them with openings on the surface of the backup sheet (11) where the absorptive body (12) is overlapped with, and these trap portions (13C) in a slit form can be formed by providing a gusset for each on the backup sheet (11) along its longitudinal direction. These trap portions (13C) in a slit form each have an opening (22) in a slit form which extends along both side edges of the absorptive body (12), making both side edges of the absorptive body (12) straddle these openings (22). At both longitudinal ends of these trap portions (13C) in a slit form, heat-sealed portions (16) are formed to prevent liquid entering the trap portions (13C) in a slit form from leaking through these sections.

The backup sheet (11) in the present example has its width-wise side edges folded toward the absorptive body (12), and is overlapped with the absorptive body (12) on its width-wise side sections. Also, a guiding sheet (23) whose ends are located inside the trap portions (13C) in a slit form is provided between the backup sheet (11) and the absorptive body (12), to guide the liquid trapped in the trap portions (13C) in a slit form to the absorptive body (12) by using the capillary phenomenon of the guiding sheet (23). The guiding sheet (23) can be made of a material of superior liquid diffusion such as tissue paper, non-woven fabric, cloth, or foamed resin film.

When this absorptive product P is in use, because the openings (22) of the trap portions (13) in a slit form will be partially open, the liquid that cannot be instantaneously absorbed by the absorptive body (12) will flow through these openings (22) into each trap portion (13C) in a slit form, causing the trap portions (13C) in a slit form to evaginate and temporarily trap the liquid there, but eventually it will be guided by the guiding sheet (23) to be absorbed and held by the absorptive body (12). This can be used repeatedly as long as some absorption capability of the absorptive body (12) remains, maximizing the use of its absorption capability.

In the present example, two trap portions (13C) in a slit form were formed on the backup sheet (11), but it is also possible, naturally, to form one or three or more than three trap portions (13C) in a slit form.

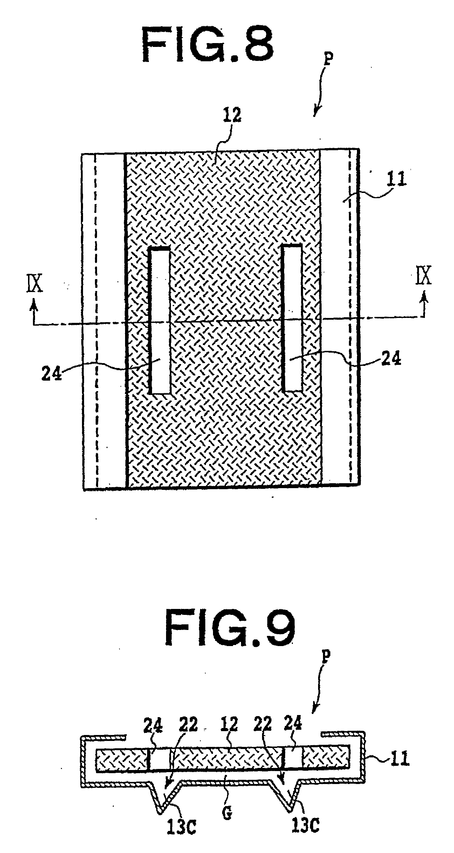

The absorptive product P shown in Fig. 8 and its sectional view taken along the line IX to IX, shown in Fig. 9, has a pair of continuous apertures (24) formed in the absorptive body (12), penetrating from the surface of the absorptive body (12) and facing the openings (22) of the trap portions (13C) in a slit form made on the backup sheet (11), to quickly guide the liquid that cannot be instantaneously absorbed by the absorptive body (12) to the trap portions (13C) in a slit form. Also, by providing a gap (G) between the backup sheet (11) and the absorptive body (12), it is designed to let the liquid that cannot be instantaneously absorbed flow easily through the gap (G) between the backup sheet (11) and the absorptive body (12) and into the trap portions (13C) in a slit form via the openings (22).

As a method of forming the gap (G) between the backup sheet (11) and the absorptive body (12), folding flat, alternately in zigzag, every other or every two, for instance, of the strips which form the exposed base sheet (18) in the absence of the highly absorbent layer (19) of the absorptive body (12S) in a sheet form shown in Fig. 4, to give bulkiness to the absorptive body (12C); or adding in between the backup sheet (11) and the absorptive body (12) a resin film with multiple openings, and each of them in a protruded state, will realize a gap. In this case, even when bulkiness is added to the absorptive body by folding the absorptive body (12S) in a sheet form, its thickness can be maintained far thinner than a conventional absorptive body that forms the acquisition layer or the transfer layer.

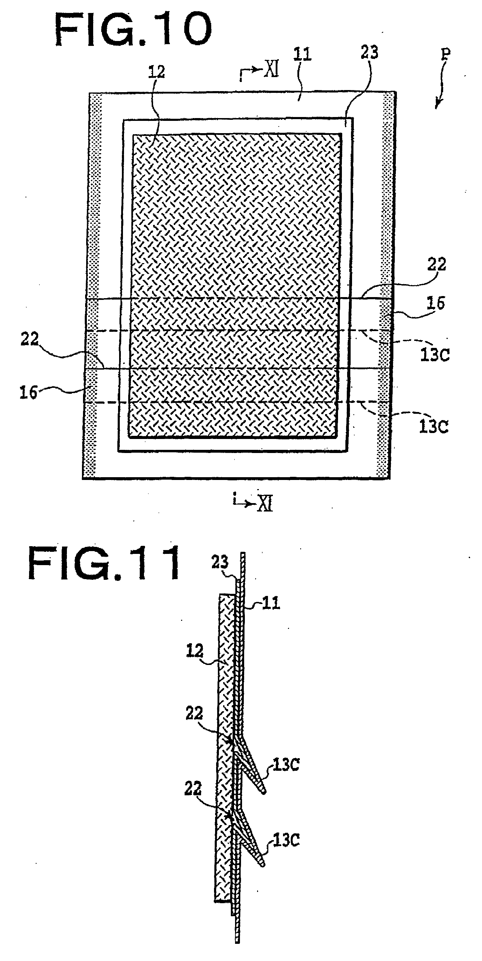

In the example stated above, the trap portions (13C) in a slit form were extended along the absorptive body (12) in a longitudinal direction, but it is also possible to let it extend along the absorptive body (12) in a width-wise direction. Fig. 10 and its sectional view taken along the line XI to XI shown in Fig. 11 indicate another example of the absorptive product P in accordance with the present invention, in which the trap portions (13C) in a slit form are formed along the absorptive body (12) in a width-wise direction with their openings on the surface of the backup sheet (11) with which the absorptive body (12) is overlapped. The trap portions (13C) in a slit form of the present example can be formed by adding gussets to the backup sheet (11) along the width-wise direction of the backup sheet (11), and the guiding sheet (23) is integrally joined to this backup sheet (11) to guide the liquid trapped in the trap portions (13C) in a slit form to the absorptive body (12). In this example, two trap portions (13C) in a slit form were made on one end (bottom end in the Fig.) of the absorptive body (12) with some space between the two; but it is also possible, naturally, to make one or three or more trap portions (13C) in a slit form on the backup sheet (11).

The absorptive product P of the present invention has the above-mentioned examples as its basic components, and the desired type of use can be obtained by adding or changing the functions, depending on the use. The location of the pouched trap portion (13B) and the trap portions (13C) in a slit form in the absorptive product P needs to be determined cautiously by considering conditions such as the absorptive product P's shape, size, use location, posture of the wearer and so on, and it is also possible, naturally, to make the pouched trap portion (13B) or the trap portions (13C) in a slit form in a location other than those in the above-mentioned examples. In addition, the absorptive product P that has, as its trap portion, the trap portions (13C) in a slit form only, is especially suited for open-type or pants-type diapers for babies or adults.

The absorptive product P of the present invention will be sequentially explained per specific use, but as for the functional elements identical to those of the examples already treated, they will only be marked with identical codes and the redundant explanations will be omitted.

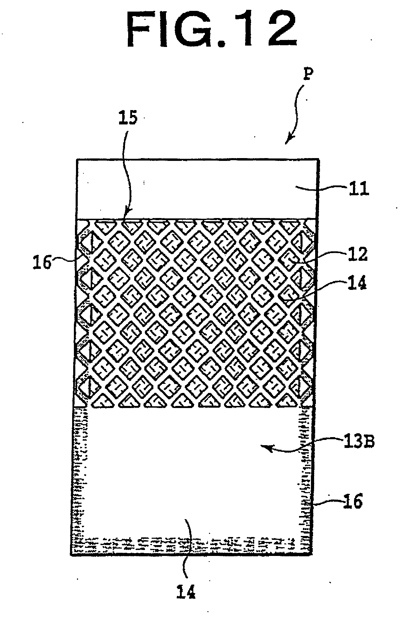

Fig. 12 shows an example of the application of the absorptive product P in accordance with the present invention to a female continence pad or urine pad. The present example uses a trap sheet (14) whose top half is made of a mesh sheet to allow free entering and exiting of the liquid and whose lower half is made liquid-impermeable, which makes it possible to treat a large amount of urine by temporarily guiding any amount of urine that is more than what the absorptive body (12) can absorb to this area. Also, this example has a structure that allows the absorptive body (12) to be put in and taken out by forming an opening (15) in the pouched trap portion (13B), but it is also possible to make it impossible to take out the absorptive body (12), by joining the outer margin of the trap sheet (14) entirely to the trap sheet (11).

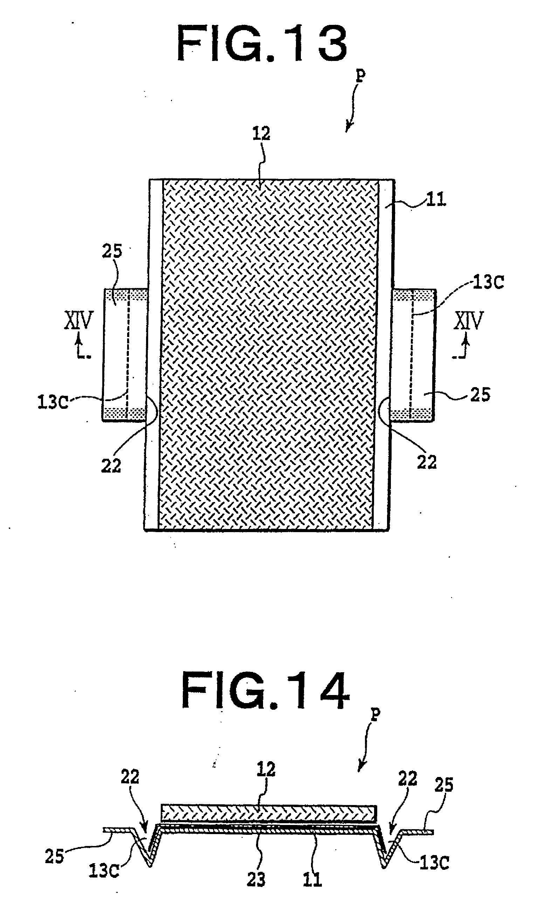

The absorptive product P shown in Fig. 13 and its sectional view Fig. 14 taken along the line XIV to XIV is also suitable for use in light-case to medium-case continence pads, especially for women. A pair of flaps (25) extending in a width-wise direction from both width-wise side edges of the absorptive body (12) are formed on the backup sheet (11), and a trap portion (13C) in a slit form is formed on each of the base end of the flaps (25). Between the backup sheet (11) and the absorptive body (12) the guiding sheet (23) whose both ends are located inside the pair of trap portions (13C) in a slit form, on right and left, is interpositioned to guide the liquid that is temporarily trapped in the trap portions (13C) in a slit form to the absorptive body (12) by using the capillary phenomenon of this guiding sheet (23).

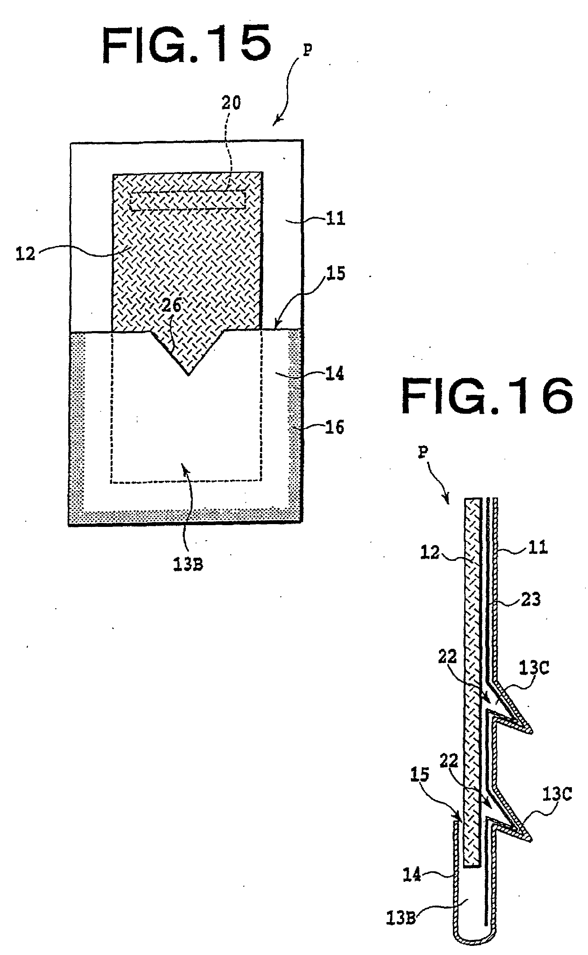

Fig. 15 shows an example of the application of the absorptive product P of the present invention to a men's continence pad. The present example has the penis-guiding section formed on the trap sheet (14) to guide the penis into the pouched trap portion (13B), with a crena in a V-canaliform (26) formed in the center of the top end of the trap sheet (14).

The absorptive product P in accordance with the present invention, shown in Fig. 16, is a combination of the examples shown in Fig. 1, Fig. 10 and Fig. 11, and by forming the pouched trap portion (13B) and the trap portions (13C) in a slit form like these in a single absorptive product P, temporarily holding of further larger volumes of liquid becomes possible, and it will be suitable for use as urine pads, especially for men.

For any of the examples stated above, the liquid-impermeable backup sheet (11) or trap sheet (14) must be employed, but when the flexible liquid-impermeable inner bag is incorporated in the pouched trap portion (13B), as the outer bag of the present invention, the liquid-permeable material can be used as the trap sheet (14).

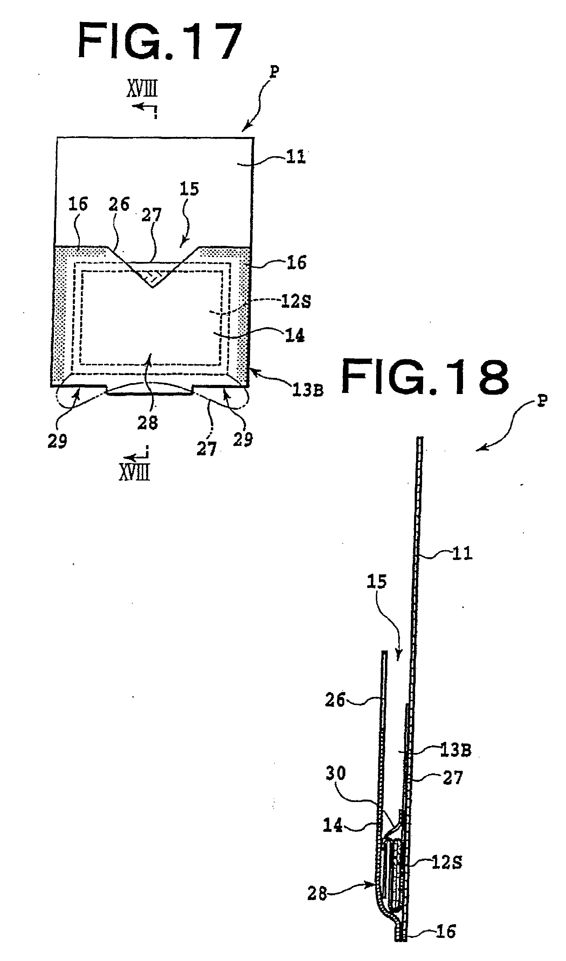

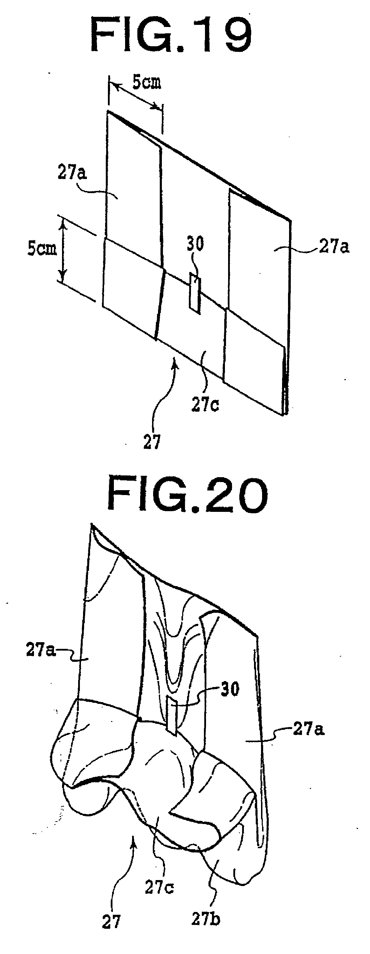

Fig. 17 and Fig. 18, that shows a sectional view taken along the line XVIII to XVIII, depict an example in which an inner bag (27) like this is incorporated and, in this example, the entire absorptive body (12) is held in the inner bag (27). On both left and right width-wise edges on the bottom end of the outer bag (28) that forms, along with the inner bag (27), the pouched trap portion (13B), a crena (29) on each edge is formed in such a manner that it spreads over the outside and inside of the outer bag (28), and when the urine accumulates in the flexible inner bag (27), this inner bag (27) is designed to expand and portions of it to evaginate through the crenas (29), as shown in the chain double-dashed line in Fig. 17, thus preventing the urine from overflowing from the inner bag (27) to the outer bag (28).

The external appearance of the inner bag (27) of the present example is shown in Fig. 19 and its external appearance in the state where the urine is temporarily accumulated is illustrated in Fig. 20. The inner bag (27) in the present example is made of Sanipak Company of Japan Ltd.'s PE film in a rectangular shape whose dimensions are, for instance, 28 cm in length, 24 cm in width and 0.02 mm in thickness, where the first folded portion (27a) is formed by folding inward the longer side by 5 cm each on both the left and right sides, and then the second folded portion (27b) is formed by folding upward the shorter side by 10 cm, and furthermore the third folded portion (27c) is formed by folding downward this second folded portion (27b). The center of the third folded portion (27c) and, located above it, the center of the top part on the shorter side end are joined on each end of the hanger tape (30).

When urine is discharged into the inner bag (27), therefore, the inner bag (27) expands inside the outer bag (28) and its first and second folded portions (27a, 27b) located on each side of the hanger tape (30) evaginate due to gravity, then these portions are designed to evaginate through each crena (29) formed in the outer bag (28). In this way, even if a large volume of urine is discharged, urine-leakage inside the outer bag (28) can be prevented.

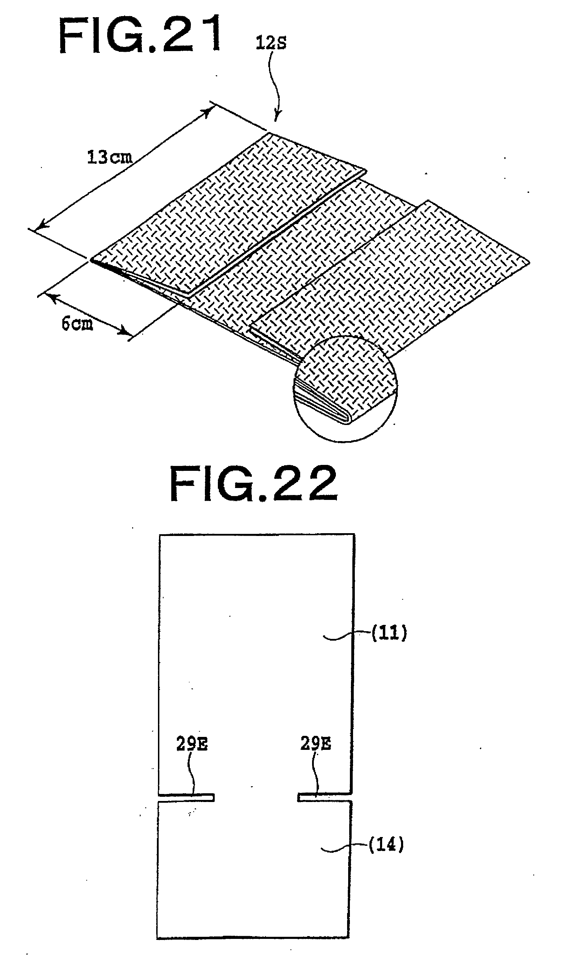

The external appearance of the absorptive body (12) of the present example, which is held in the inner bag (27), is shown in Fig. 21. That is, the absorptive body (12) of the present example is the absorptive body in sheet-form (12S), as shown in Fig. 4, whose trade name is MegaThin (Registered Trademark), made by Japan Absorbent Technology Institute, in a rectangular shape whose dimensions are, for instance, 28 cm in length and 26 cm in width, and with a folded construction formed by first folding its shorter side in half, and then by further folding inward on both left and right sides of it by 6cm.

The backup sheet (11) and the trap sheet (14) of this absorptive product P use, as shown in Fig. 22, the non-woven fabric (Type S.M.M.S.) made by Avgol, in a rectangular shape whose dimensions are, for instance, 38 cm in length, 20 cm in width and 20g/m² basis weight, and where the trap portion is formed by making a 7-cm-long crena (29E) each on both sides of the sheet at 14 cm from the longitudinal end of the sheet, then by folding the portion that makes the trap sheet (14) at these crenas (29E), and then by forming the heat-sealed portion (16) on both width-wise side edges as well as on the right and left sides of the top edge of the folded trap sheet. In this case, prior to forming the heat-sealed portion (16) on the right and left sides of the top edge of the trap sheet (14), the inner bag (27) and the absorptive body (12) must be incorporated inside the outer bag (28). Although in the present example, the absorptive body (12), after it is folded, is held in its entirety inside the inner bag (27), the expandability of the absorptive body (12) can be maximized when only one end of it is held inside the inner bag (27), as shown in the previous examples.

While the above-mentioned absorptive product P can be used as it is by combining it with the conventional disposal diaper or underpants including shorts and pants, dedicated underpants with a fitting section that can hold this absorptive product P in the prescribed location are also a preferable mode for carrying out the present invention.

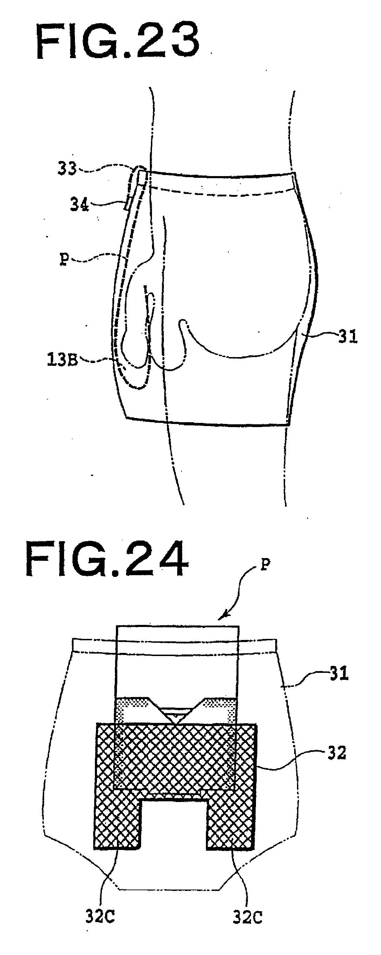

Fig. 23 through Fig. 26 show each of such examples of the underpants in accordance with the present invention, and it is preferable that all these underpants are made of plain knit material such as jersey knit to ensure excellent fit to the skin when worn.

Fig. 23 shows the urine pad, also shown in Fig. 17 through Fig. 21, or the underpants (31), designed to suit the absorptive product P, in the state of being put on a wearer, and Fig. 24 is an illustrative drawing of its inner structure. The mesh pocket (32) with escapes (32C) to accommodate the portions of inner bag (27) that can evaginate through the crenas (29) is sewn on inside the underpants (31), as a fitting section to fit the product of the present invention. The external front body of these underpants (31) has a joining point (34) to detachably join the fixing tape (33) joined in advance to the other end of the absorptive product P's backup sheet (11), which ensures stable retention of the absorptive product P inside the underpants (31) when worn, and thus prevent dislocation of it. For the fixing tape (33), for instance, one with a hook and loop fastener placed on each of the joining surfaces can be adopted, while the hook and loop fasters matching these fasteners are placed at the joining points (34) on the underpants (31).

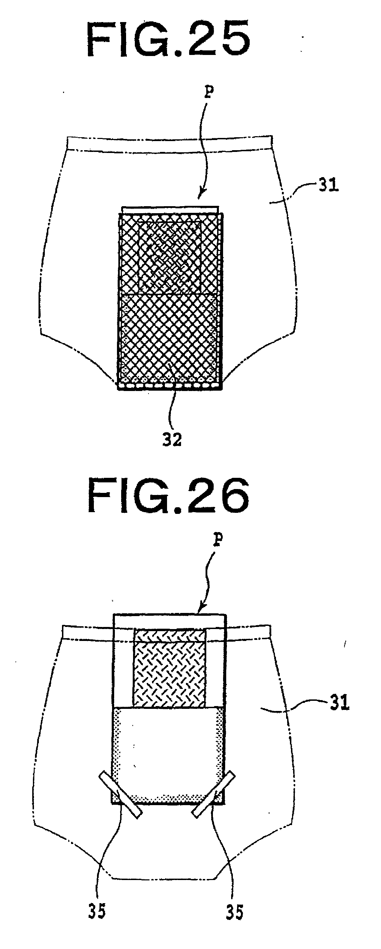

The underpants (31) in the example shown in Fig. 25 have a vertically long mesh pocket (32), that can hold the absorptive product P shown in Fig. 15, sewn on the inside of the underpants (31).

The underpants (31) in the example shown in Fig. 26 have a pair of elastic members (35) sewn in an inclined state on the inside of the underpants (31), to hold both the right and left sides of the bottom end of the absorptive product P shown in Fig. 1.

As described above, the structure and configuration of the fitting section used to put the absorptive product on may be selected according to the type of use and other conditions. And at the same time, the joining points (34) placed on the front body outside the underpants (31) may also be changed depending on the configuration of the fixing tape (33).

An experiment to verify the absorption effectiveness by actually putting on the absorptive product P (urine pad) shown in Fig. 17 through Fig. 21 was conducted. The basic characteristics of the absorptive body (12) used for the experiment are as follows:

| | |
|---|---|
| Basis Weight of the SAP | 150g/m² |
| Estimated Absorbed Volume | 55ml/g |
| Estimated Retention | 35ml/g |

The estimated absorbed volume of the entire absorptive body (12), therefore, is 400ml and the estimated retention 260ml.

The underpants (31) shown in Fig. 24, the L-size Cotton Trunks (Trade Name) made by Gunze Limited were put on a male test subject person, and then he was asked to urinate with the underpants on. 30 seconds after his urinating, the inner bag (27) was expanded to the maximum, and a portion of it evaginated through the crenas (29), but no leakage of urine from the inner bag (27) to the outer bag (28) was observed. Next, 3 minutes after he started urinating, this absorptive product P was collected from the test subject person to measure the amount absorbed by the absorptive body (12). As a result, absorption of 430ml of urine was confirmed. This absorbed volume far exceeds the estimated retention of 260ml, as well as the estimated absorbed volume of 400ml. With this, it was verified that extremely effective absorption took place.

In the above-mentioned examples, the ones that incorporate in the absorptive products the trap portions that temporarily trap the liquid were described, but the ones that have these trap portions provided in the underpants themselves are also included in the present invention.

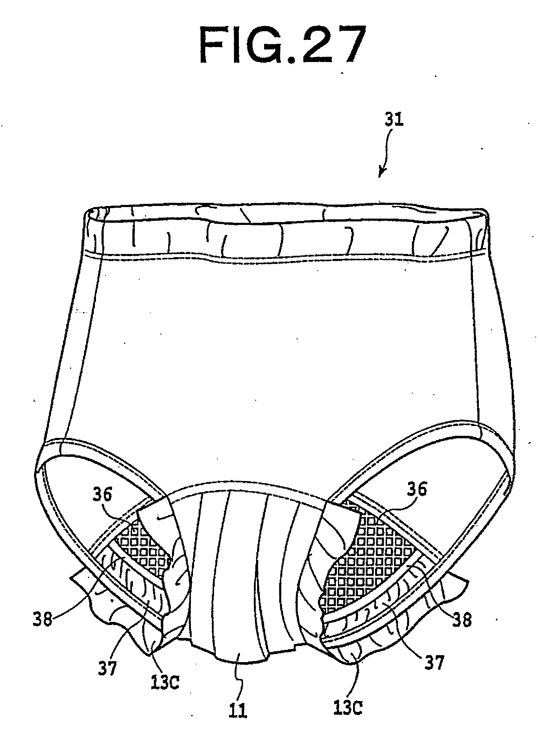

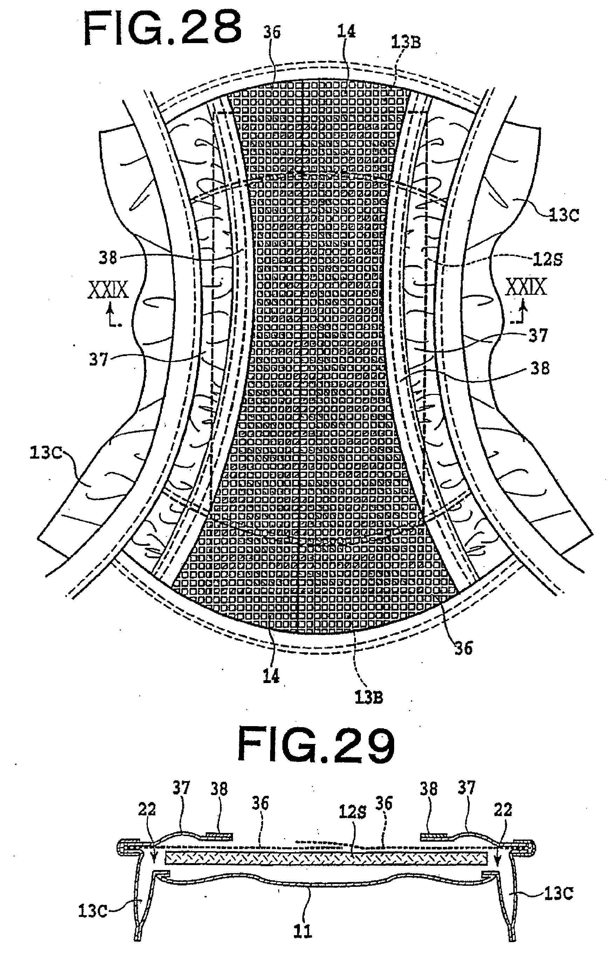

Fig. 27 shows the external appearance of the example of the application of these underpants in accordance with the present invention to the women's panties, while Fig. 28 shows the planar shape of their crotch, and Fig. 29 shows the section view taken along the line XXIX to XXIX. That is, the crotch of these underpants (31) is made of a liquid-impermeable backup sheet (11) and the rest is made of jersey knit of material such as cotton to ensure comfort for the wearers. In case it is impossible to obtain sufficient strength of the backup sheet (11), however, it is also effective to make the entire underpants (31) with jersey knit of material such as cotton and then line the crotch with the backup sheet (11). On both right and left side edges of this backup sheet (11) are a pair of the trap portions (13C) in a slit form with an opening (22) each, and that extend along the right and left side edges of the crotch treated with piping, and these make up the section to fit the present invention. On both fore and aft ends of the crotch, the liquid-impermeable trap sheet (14) is placed, and the outer margin, excluding the end edges facing the center of the crotch, is joined to the backup sheet (11), forming the pouched trap portions (13B) between the trap sheets and the backup sheet (11). Furthermore, the outer margin of a pair of mesh sheets (36), on the right and left, overlapping with these trap sheets (14) and the backup sheet (11), is sewn in the crotch, and the inner edges of these are joined to the trap sheet (14) in such a manner that these end edges overlap each other. A pair of liquid-impermeable side sheets (37) forming the standing gathers on the right and left sides of the crotch are incorporated, and the elastic member (38) is applied in the stretched state on the inner edges of these.

Therefore, when the absorptive body (12) is fitted to these underpants (31), in order to make both ends in a longitudinal direction of the absorptive body (12) held in the pouched trap portions (13B), the overlapped section of the mesh sheet (36) between a pair of trap sheets (14) is opened to insert the absorptive body (12) there and have it held in the crotch. Because the absorptive body (12) is detachable from these underpants (31), by replacing the absorptive body (12) as required, these underpants (31) can be used repeatedly. In this case, as the absorptive body (12), the absorptive body (12S) in a sheet form, shown in Fig. 4, can be used as it is.

The urine that cannot be instantaneously absorbed by the absorptive body (12) when in use is temporarily held in the pouched trap portions (13B) and the trap portions (13C) in a slit form, and is absorbed by the absorptive body (12) gradually over time, enabling an effective absorption of an extremely large amount of urine.

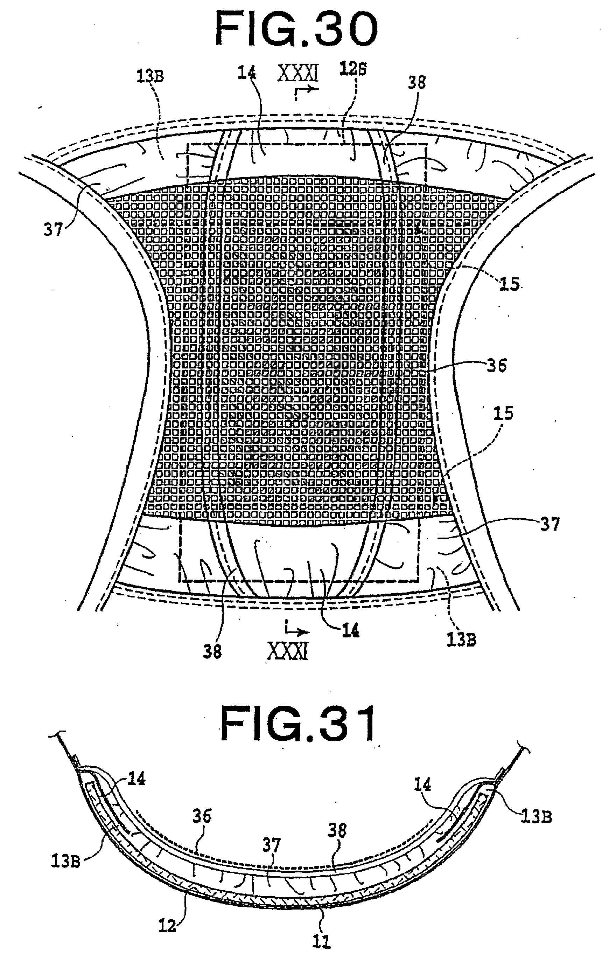

In case the underpants like these are applied to men's briefs, it is preferred that the location of the pair of trap portions on right and left as mentioned above is changed. For instance, the planar shape of the crotch of the example where the underpants of the present invention are applied to men's briefs is shown in Fig. 30 and its section view taken along the line XXXI to XXXI is shown in Fig. 31. That is, the crotch of the underpants (31) entirely made of cotton jersey knit is lined with the liquid-impermeable backup sheet (11); the liquid-impermeable trap sheet (14) is placed on both fore and aft ends of the crotch; and the outer margin, excluding the end edges facing the center of the crotch, is joined to the backup sheet (11), forming the pouched trap portions (13B), or the fitting section of the present invention, along with the backup sheet (11). A pair of liquid-impermeable side sheets (37) forming the standing gathers on the right and left sides of the crotch are incorporated, and the elastic member (38) is applied in a stretched state on the inner edges of these. In the present example as well, in order to prevent the absorptive body (12) from directly contacting the wearer's skin, the right and left ends of the mesh sheet (36), overlapping with the backup sheet (11) exposed between a pair of pouched trap portions (13B), are joined to the right and left side edges of the crotch.

Therefore, in order to make both ends in a longitudinal direction of the absorptive body (12) held in the pouched trap portions (13B), the absorptive body (12), such as the one shown in Fig. 4, can be inserted through the longitudinal end of the mesh sheet (36), and held in the crotch. Because the absorptive body (12) is detachable from these underpants (31), by replacing the absorptive body (12) as required, these underpants (31) can be used repeatedly.

As in the previous examples, the urine that cannot be instantaneously absorbed by the absorptive body (12) is temporarily held in the trap portions (13C) in a slit form, located on the lower side in the direction of gravitational force, and will be absorbed by the absorptive body (12) gradually over time.

### Industrial Applicability

In accordance with the absorptive product of the present invention and the underpants using the same, as well as inventions incidental to these, the extremely thin and non-bulky absorptive products are provided. The underpants using the same are improved in comfort for the wearers and therefore, they have a great deal of potential in industry.

## Claims

1. An absorptive product, **characterized in that** it comprises an absorptive body that can absorb and retain a liquid and a flexible and liquid-impermeable trap portion to temporarily trap the liquid that is to be absorbed by this absorptive body, and that at least a part of the said absorptive body and the said trap portion are made to overlap.

2. The absorptive product, according to claim 1, **characterized in that** the said trap portion is formed with a liquid-impermeable sheet, and the surface of this sheet and at least a part of the said absorptive body are made to overlap.

3. The absorptive product, according to claim 2, **characterized in that** the said trap portion has an opening facing the surface of the said sheet.

4. The absorptive product, according to claim 3, **characterized in that** the said opening is extended along the said absorptive body in a longitudinal direction.

5. The absorptive product, according to claim 3, **characterized in that** the said opening is extended along the said absorptive body in a width direction.

6. The absorptive product, according to any one of claims 3 through 5, **characterized in that** the said absorptive body is positioned to straddle the said opening.

7. The absorptive product, according to claim 6, **characterized in that** the said absorptive body has a continuous aperture from the opposite side of the surface of the said sheet, facing the said opening of the said trap portion.

8. The absorptive product, according to any one of claims 2 through 4, **characterized in that** the said sheet has a pair of flaps extended along both width-directional side edges of the said absorptive body, and the said trap portion is formed in each of this pair of flaps.

9. The absorptive product, according to any one of claims 1 through 4, **characterized in that** the said trap portion is positioned on both width-directional sides of the said absorptive body.

10. The absorptive product, according to any one of claims 1 through 4, **characterized in that** a guiding member to guide the liquid trapped in the said trap portion to the said absorptive body is further provided.

11. The absorptive product, according to claim 1, **characterized in that** the said trap portion is formed with a liquid-impermeable sheet, and has a penis-guiding section to guide the penis into it, while at least one end of the said absorptive body is held in the said trap portion.

12. The absorptive product, according to claim 1, **characterized in that** the said trap portion has an outer bag, with a penis-guiding section to guide the penis into it, and a flexible and liquid-impermeable inner bag that is incorporated in this outer bag and holds at least one end of the said absorptive body.

13. The absorptive product, according to claim 12, **characterized in that** the said inner bag has a folded portion that can evaginate.

14. The absorptive product, according to claim 13, **characterized in that** the said outer bag further has an opening to allow parts of the said folded portion of the said inner bag to evaginate out of the said outer bag.

15. The absorptive product, according to any one of claims 2 through 14, **characterized in that** a dislocation prevention member is further provided between the said absorptive body and the said sheet, to prevent the said absorptive body from dislocating against the said sheet.

16. The absorptive product, according to any one of claims 2 through 15, **characterized in that** a pair of elastic members are further provided along the right and left side edges of the said sheet, joined in a stretched state to both the right and left side edges of this applicable sheet.

17. The absorptive product, according to any one of claims 1 through 16, **characterized in that** the said absorptive body has a non-woven substrate in a sheet form and multiple liquid-absorbing portions coated on this non-woven substrate at prescribed intervals.

18. The absorptive product, according to any one of claims 1 through 17, **characterized in that** the said absorptive body contains 50 to 95 % by weight of SAP.

19. The absorptive product, according to any one of claims 1 through 18, **characterized in that** the said absorptive body has a hydrodisintegrative characteristic.

20. A method of producing an absorptive product, according to claim 14, **characterized in that** the method of producing the absorptive product involves a step to form a pair of width-directional straight-line crenas along both side edges of the single sheet material to make an opening on each side of this single sheet material, a step to fold the said single sheet material in the width direction in such a manner to include the said pair of crenas in the folded portion, and a step to form a trap portion by joining the overlapping width-directional side edges of the said folded single sheet material.

21. An inner bag used for an absorptive product, according to claim 13, **characterized in that** it has a folded portion comprising a first pair of folded portions, one each on the right and left, formed by folding the right side and left side of the rectangular sheet toward the center, a second folded portion formed by folding the bottom end of the said sheet upward, a third folded portion formed by folding the top end of this second folded portion downward, and a hanger tape each of whose both ends is joined to the center of this third folded portion and the center of the top part of the said sheet.

22. A method of producing an inner bag, according to claim 21, **characterized in that** it has a step to form a first pair of folded portions, one each on the right and left, by folding the right side and left side of the rectangular sheet toward the center, a step to form a second folded portion by folding the bottom end of the said sheet upward, a step to form a third folded portion by folding the top end of this second folded portion downward, and a step to join both ends of a hanger tape each to the center of this third folded portion and the center of the top part of the said sheet.

23. Underpants, **characterized in that** they have a fitting section to which an absorptive product according to any one of claims 1 through 19 or an absorptive product produced according to claim 20 is fitted, in such a manner that it is detachable.

24. Underpants, **characterized in that** they have an absorptive product according to any one of claims 1 through 19 or an absorptive product produced according to claim 20 and a fitting section to which this absorptive product is fitted, in such a manner that it is detachable.

25. The underpants, according to claim 23 or 24, **characterized in that** the said fitting section is provided at least inside the said underpants.

26. The underpants, according to any one of claims 23 through 25, **characterized in that** the said underpants are of a plain knit product.

27. Underpants, **characterized in that** they have an absorptive body holding section to detachably hold the absorptive body that can absorb and retain a liquid and a flexible and liquid-impermeable trap portion to temporarily trap the liquid that is to be absorbed by this absorptive body.

28. Underpants, **characterized in that** they have an absorptive body that can absorb and retain a liquid, an absorptive body holding section to detachably hold this absorptive body and a flexible and liquid-impermeable trap portion to temporarily trap the liquid that is to be absorbed by this absorptive body.

29. The underpants, according to claim 27 or 28, **characterized in that** the said trap portion has an opening facing the inner surface of the said underpants.

30. The underpants, according to claim 29, **characterized in that** the said opening is extended along the said absorptive body in a longitudinal direction.

31. The underpants, according to any one of claims 27 through 30, **characterized in that** the said trap portion is positioned on both sides of the crotch portion of the underpants.

32. The underpants, according to claim 27 or 28, **characterized in that** the said trap portion has an opening into which at least one end of the said absorptive body is inserted.

33. The underpants, according to any one of claims 27 through 32, **characterized in that** the said absorptive body has a non-woven substrate in a sheet form and multiple liquid-absorbing portions coated on this non-woven substrate at prescribed intervals.

34. The underpants, according to any one of claims 27 through 33, **characterized in that** the said absorptive body contains 50 to 95 % by weight of SAP.

35. The underpants, according to claim 28, **characterized in that** a backup sheet and a guiding sheet are further provided, the said guiding sheet is positioned between the said absorptive body and the said backup sheet, at least one end of the said guiding sheet is located inside the said trap portion, and urine is trapped in the said trap portion and is guided by the said guiding sheet to the said absorptive body, to be absorbed by the said absorptive body.
